Europäisches Patentamt

European Patent Office

Office européen des brevets

(11) Veröffentlichungsnummer: **0 308 646**
**A2**

(12) **EUROPÄISCHE PATENTANMELDUNG**

(21) Anmeldenummer: **88113233.6**

(22) Anmeldetag: **12.08.88**

(51) Int. Cl.⁴: **C07C 53/126 , C07C 51/41**

(30) Priorität: **23.09.87 DE 3731919**

(43) Veröffentlichungstag der Anmeldung:
**29.03.89 Patentblatt 89/13**

(84) Benannte Vertragsstaaten:
**AT BE CH DE ES FR GB GR IT LI LU NL SE**

(71) Anmelder: **Giulini Chemie GmbH**
**Giulinistrasse 2 Postfach 150 480**
**D-6700 Ludwigshafen/Rhein(DE)**

(72) Erfinder: **Martin, Roland, Dr. Dipl.-Ing**
**Kastanienweg 21**
**D-6940 Weinheim(DE)**
Erfinder: **Schanz, Klaus, Dr. Dipl.-Chem.**
**In der Zeil 5**
**D-6701 Dannstadt-Schauernheim(DE)**
Erfinder: **Kaufmann, Bruno**
**Ormsheimerhof 16**
**6710 Frankenthal(DE)**

(74) Vertreter: **Benatzky, Erika, Dr.**
**Giulinistrasse 2 Postfach 150480**
**D-6700 Ludwigshafen/Rh.(DE)**

(54) **Neue Aluminium-Magnesium-Hydroxi-Verbindungen.**

(57) Die Erfindung betrifft neue Aluminium-Magnesium-Hydroxi Verbindungen, ihre Herstellung und ihre Verwendung als Verdickungs-, Thixotropierungs-, Stabilisierungs-und Antiabsetzmittel z.B. für Farben,Lacke,Klebstoffe und Kosmetika.
Die neuen Verbindungen enthalten einen organischen Monocarbonsäurerest RCOO⁻ mit 2 bis 22 Kohlenstoffatomen und weisen eine Schichtstruktur auf.

EP 0 308 646 A2

## Neue Aluminium-Magnesium-Hydroxi-Verbindungen

Die vorliegende Erfindung betrifft neue Verdickungs-, Thixotropierungs-, Stabilisierungs- und Antiabsetzmittel, zum Beispiel für Farben, Lacke, Anstrichmittel, Kitte, Wachse, Klebstoffe und Kosmetika, auf Basis von Aluminium-Magnesium-Hydroxi-Verbindungen sowie ein Verfahren zu ihrer Herstellung.

Als Verdickungs- und Thixotropierungsmittel werden auf den beispielhaft genannten Einsatzgebieten schon seit langem organophile Schichtsilikate, wie organophile Montmorillonite, Hectorite und Bentonite eingesetzt, die entstehen, wenn man quellfähige Schichtsilikate mit polaren, langkettigen, organischen Molekülen belegt, insbesondere mit quartären Ammoniumverbindungen. Gelbildende organophile Schichtsilikate sind bereits Gegenstand zahlreicher Schutzrechte. So werden in EU-A1 0 204 240 organophile Schichtsilikate beschrieben, deren austauschbare Kationen ganz oder teilweise durch quartäre Ammoniumverbindungen ersetzt sind. Die so hergestellten Schichtsilikate sind in einem organischen Lösungsmittel unter Gelbildung dispergierbar. Werden als Schichtsilikate natürliche Tonmineralien eingesetzt, ist zuvor eine Reinigung des Rohproduktes durch Abtrennung von nicht quellfähigem Material notwendig. Da das Schichtsilikat außerdem in einer hochquellfähigen Form vorliegen sollte, wird weiterhin empfohlen, diese Form, z.B. die Natriumform, durch eine vorgeschaltete Ionenaustauschreaktion herzustellen. Organophile modifizierte quellfähige Schichtsilikate, die quartäre Ammomiumverbindungen enthalten sowie seine Verwendung als Verdickungsmittel, sind auch Gegenstand der DE-OS 3145449.

In den deutschen Offenlegungsschriften 31 45 452 und 31 45 475 werden ebenfalls gelbildende, organophile Tone beschrieben. Im Gegensatz zu den vorstehend beschriebenen organophilen Schichtsilikaten sind jedoch nicht nur die Kationenaustauschstellen des Tons durch organische Kationen, beispielsweise quartäre Ammonium-, Phosphonium- oder Sulfoniumionen substituiert, sondern darüber hinaus sind in die Tone organische Kationen- organische Anionen-Komplexe eingelagert, die sich während der Umsetzung der wässrigen Tonaufschlämmung mit einer organischen anionischen und einer organischen kationischen Verbindung bilden.

Die organophilen Silikatkomplexe der DE-OSen 31 45 452 und 31 45 475 sind unter Bildung eines Geles in organischen Flüssigkeiten dispergierbar. Je nach ihrer Zusammensetzung sollen die Gele als Schmierfette, Schlämme auf Ölbasis, Bindemittel für Formsand, als Klebstoffe und Dichtungsmittel u.a. geeignet sein.

Es hat sich nunmehr aber gezeigt, daß viele der bisher hergestellten organophilen Tone den an sie gestellten Anforderungen nicht genügen. So wurde beobachtet, daß in Abhängigkeit von der Qualität des Ausgangstones Verfärbungen in den Stammpasten, das sind Pasten in einem organischen Lösungsmittel mit einem Feststoffgehalt von etwa 10 %, auftreten können, die auf vielen Anwendungsgebieten als störend empfunden werden und die Herstellung von transparenten oder absolut weißen Produkten verhindern. Darüber hinaus ist die Lagerstabilität vieler in organischen Lösungsmitteln dispergierter organophiler Schichtsilikate unbefriedigend. Dies macht sich dadurch bemerkbar, daß sich das Lösungsmittel schon nach kurzer Zeit abscheidet und bei der Lagerung der Stammpasten sich ein hartes Bodensediment bildet.

Ein großes Problem bei der Einarbeitung von organisch modifizierten Bentoniten in kosmetischen Formulierung ist die sehr nachteilig wirkende Eigenfarbe, die von leicht gelb bis braun variiert. Diese Eigenfarbe entsteht, wenn man die organisch modifizierten Bentonite in verschiedenen organischen Lösungsmitteln, wie Toluol, Cyclohexan, Siliconöl oder Paraffinöl dispergiert.

In kosmetischen Präparaten, in denen organisch modifizierte Bentonite als Verdickungs- und Thixotropierungsmittel enthalten sind, können außerdem Hautreizungen und Unverträglichkeiten auftreten. Dies wird hauptsächlich zurückgeführt auf die quartären Ammoniumverbindungen, die bei der organischen Modifizierung der Bentonite verwendet werden müssen und zwischen 30 bis 50 Gew.% in diesen enthalten sind.

Es stellte sich somit die Aufgabe, neue Verbindungen zu finden, welche die Nachteile der vorstehenden organophilen Schichtsilikate nicht aufweisen.

Überraschenderweise kann die gestellte Aufgabe mit Verbindungen der Formel

$$Al_x\ Mg_y\ OH_{35-z}\ R_z.\ n\ H_2O$$

gelöst werden, in der R den Rest R COO⁻ einer Monocarbonsäure mit 2 bis 22 Kohlenstoffatomen darstellt und die Indices x, y, z die folgenden Bedingungen erfüllen:

$3 \leqq x \leqq 9$
$4 \leqq y \leqq 13$

2

$$3 \leq z \leq 5$$
$$3x + 2y = 35$$

Die Herstellung der neuen Verbindungen erfolgt durch Umsetzung der wässrigen Suspension einer Verbindung der Formel $Al_x Mg_y(OH)_{35-z}(SO_4)_{z/2} \cdot nH_2O$, in der x, y die eben angegebene Bedeutung haben und für z gilt $3 \leq z \leq 5$, wobei $3x + 2y = 35$ ist, mit der wässrigen Suspension eines Alkalisalzes einer Monocarbonsäure, wobei der $R\ COO^-$-Rest 2 bis 22 Kohlenstoffatome enthält, unter Rühren bei Temperaturen zwischen $20°C$ bis $100°C$, bevorzugt zwischen $20°C$ und $60°C$. Vorzugsweise arbeitet man unter Scherkräfteeinwirkung auf die wässrigen Suspensionen. Unter diesen Verfahrensbedingungen ist die Umsetzung in vielen Fällen nach 2 Stunden vollständig.

Die Abtrennung des Reaktionsproduktes aus der wässrigen Suspension kann nach einem der bekannten Verfahren erfolgen, bevorzugt jedoch durch Filtration. Der Filterkuchen muß mit Wasser zur Entfernung des anhaftenden Alkalisulfates so lange gewaschen werden, bis im Waschwasser mit Bariumchlorid kein $SO_4{}^{2-}$ nachweisbar ist. Die Trocknung des Filterkuchens wird bei Temperaturen zwischen 60 bis $130°C$, bevorzugt aber bei 80 bis $110°C$ z.B. in einem Hordentrockner vorgenommen. Andere Trockenvorrichtungen sind ebenfalls anwendbar.

Bei einer anderen Trocknungsvariante wird der sulfatfreie Filterkuchen in Wasser resuspendiert und sprühgetrocknet, wobei die Eintrittstemperatur $T_E = 250$ bis $350°C$, bevorzugt $270°C$ bis $300°C$, und die Austrittstemperatur $T_A = 80°C$ bis $130°C$, bevorzugt $90°C$ bis $110°C$, beträgt.

Nach anderen Verfahrensvarianten wird zur wässrigen Suspension der Verbindung $Al_x Mg_y(OH)_{35-z}(SO_4)_{z/2} \cdot nH_2O$ das Alkalisalz einer Monocarbonsäure in fester Form zugesetzt, wobei alle anderen Verfahrensmerkmale gleich bleiben.

Die bei dem neuen Verfahren als Ausgangsstoff eingesetzten Al, Mg Verbindungen sind aus dem Stand der Technik bereits bekannt, z.B. aus der DE 34 08 463 C2. Die Monocarbonsäuren sind im Handel erhältliche Verbindungen. Die Herstellung der Alkalisalze kann gemäß Beispielen erfolgen.

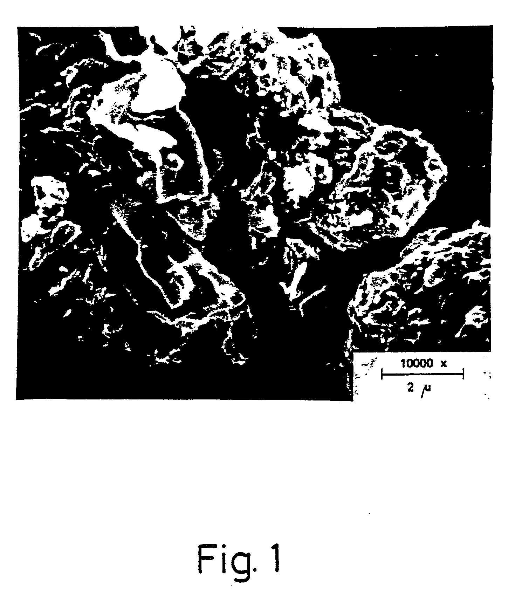

Die neuen Verbindungen sind feste, weiße und geruchlose, kristalline Stoffe. Die Charakterisierung ihrer Struktur geschieht mit Hilfe der Röntgendiffraktometrie und der Rasterelektronenmikroskopie. Durch die Röntgen diffraktometer-Aufnahmen konnte bewiesen werden, daß die neuen Verbindungen kristallin sind. Die Schichten- oder Lamellenstruktur geht aus der beiliegenden REM-Aufnahme hervor. Die Abbildung 1 zeigt die REM-Aufnahme des Produktes aus Beispiel 10.

Im Vergleich mit einem organisch modifizierten Hectorit oder Natrium-Bentonit, die beide im Handel erhältlich sind, zeigen die neuen Verbindungen einen deutlich höheren Weißgrad. Der Weißgrad ist ein Maß für die Farbe der Substanzen und wird z.B. mit dem Farbmeßgerät Tricolor LFM 3 von Dr. Lange gegen einen Emaille-Weißstandard gemessen. In der Tabelle 1 ist der Weißgrad der Produkte aus den nachstehenden Beispielen 6 - 17 sowie der Weißgrad von zwei Handelsprodukten angegeben. Aus dieser Tabelle geht es deutlich hervor, daß die neuen Verbindungen einen deutlich höheren Weißgrad besitzen, also fast weiß sind, während die Handelsprodukte gefärbt sind.

Tabelle 1 : Vergleich der Weißgrade

Produkt aus Beispiel 6:     98,1
Produkt aus Beispiel 7:     98,0
Produkt aus Beispiel 8:     98,1
Produkt aus Beispiel 9:     98,2
Produkt aus Beispiel 10:    97,9
Produkt aus Beispiel 11:    98,4
Produkt aus Beispiel 12:    98,3
Produkt aus Beispiel 13:    98,2
Produkt aus Beispiel 14:    98,1
Produkt aus Beispiel 15:    98,3
Produkt aus Beispiel 16:    98,4
Produkt aus Beispiel 17:    98,1
Natrium-Bentonit      91,3
Org. modif. Hectorit      91,8

Die Wirksamkeit des neuen Produktes als Antiabsetzmittel schon in Konzentration von 2 % zeigen die nachstehenden Versuche:

3

Es wurden Formulierungen, wie in Tabelle 2 beschrieben, hergestellt und die Absetzkurve über eine Trübungsmessung mit Hilfe eines Eppendorf-Photometers bestimmt:

In einem 300 ml Becherglas wird die Extinktion des Lösungsmittels Paraffinöl auf 0 eingestellt, d.h. 100 % Durchlässigkeit. In das gleiche Becherglas werden nun die Produkte aus den einzelnen Beispielen im Vergleich mit im Handel erhältlichem Na-Bentonit und einem org. mod. Hectorit in 2 %iger Konzentration in Paraffinöl gegeben, durch Rühren homogen suspendiert und danach 3 Min. lang mit 100 U/min. gerührt. Der Rührer wird abgestellt und die Abnahme der Extinktion über einen Schreiber verfolgt. Der Extinktionswert, den man direkt nach dem Abstellen des Rührers erhält, wird mit 0 % Durchlässigkeit angenommen.

Aus der Tabelle 2 kann man leicht erkennen, daß die in der Erfindung hergestellten Produkte sich wesentlich schwerer als die Vergleichsprodukte absetzen, was ein Vorteil bei Pigment-Farben ist.

Tabelle 2

| Absetzversuche (% Durchlässigkeit) | | | | | |
|---|---|---|---|---|---|
| Produkt aus Beispiel | 1 h | 2 h | 3 h | 6 h | 8 h |
| 6 | 0,2 | 0,4 | 1,0 | 11,9 | 21,0 |
| 7 | 0,8 | 1,2 | 1,4 | 4,7 | 9,0 |
| 8 | 1,0 | 2,0 | 2,8 | 13,6 | 23,5 |
| 9 | 0,8 | 0,8 | 1,2 | 4,7 | 8.5 |
| 10 | 0,8 | 0,8 | 0,8 | 3,5 | 7,0 |
| 11 | 0,8 | 0,8 | 1,2 | 2,5 | 5,8 |
| 12 | 0,4 | 0,4 | 0,4 | 1,6 | 5,5 |
| 13 | 0,2 | 0,2 | 0,2 | 1,5 | 5,3 |
| 14 | 0,2 | 0,2 | 0,3 | 1,5 | 5,0 |
| 15 | 0,2 | 0,2 | 0,2 | 1,6 | 5,4 |
| 16 | 0,4 | 0,6 | 0,8 | 2,0 | 6,1 |
| 17 | 0,6 | 0,6 | 1,0 | 2,5 | 6,3 |
| Na-Bentonit: | 2,3 | 7,6 | 12,5 | 33,0 | 51,0 |
| Org. mod. Hectorit: | 2,0 | 6,5 | 9,0 | 22,2 | 33,0 |

Separat wird hierzu das Absetzvolumen nach verschiedenen Zeiten in einem 100 ml Meßzylinder bestimmt. Dazu wird die Formulierung 20mal vertikal und 20mal horizontal geschüttelt und dann stehen gelassen. Das Absetzvolumen gibt Auskunft über die leichte Dispergierbarkeit der organischen Produkte der Erfindung in diesem Lösungsmittel unter geringen Scherkräften.

Im Unterschied zu den Absetzversuchen aus den Tabellen 2/3 wurden hier die Produkte nach der Erfindung (2 %) mit einem in diesem Lösungsmittel unlöslichen Stoff (z.B. Aluminiumchorohydrat mit einer Korngröße von 90 % im Bereich zwischen 10 und 75 μm) in 15 %iger Konzentration in dem jeweiligen Lösungsmittel suspendiert. Man kann auch erkennen, daß sich die suspendierten Produkte bei Zugabe der Substanzen aus den Beispielen 6 - 11 schwerer absetzen.

Tabelle 3

| Absetzvolumen (ml) in Siliconöl (Typ 345 von Dow Corning) | | | | | | | | | |
|---|---|---|---|---|---|---|---|---|---|
| Produkt aus | 0,5 min | 1 min | 3 min | 7 min | 10 min | 20 min | 30 min | 60 min | 2 h | 14 h |
| Beispiel 6 | 99 | 98 | 98 | 91 | 90 | 80 | 75 | 65 | 53 | 23 |
| Beispiel 7 | 100 | 100 | 99 | 98 | 97 | 93 | 89 | 74 | 58 | 24 |
| Beispiel 8 | 100 | 100 | 98 | 96 | 95 | 90 | 85 | 72 | 56 | 24 |
| Beispiel 9 | 100 | 99 | 98 | 96 | 94 | 88 | 82 | 77 | 61 | 27 |
| Beispiel 10 | 100 | 99 | 98 | 96 | 94 | 89 | 81 | 70 | 54 | 27 |
| Beispiel 11 | 100 | 99 | 97 | 94 | 92 | 84 | 78 | 67 | 54 | 25 |
| ohne Zusatz | 98 | 95 | 73 | 44 | 40 | 35 | 32 | 30 | 25 | 25 |
| Na-Bentonit: | 100 | 99 | 97 | 82 | 68 | 47 | 43 | 38 | 35 | 25 |
| Org.mod. Hectorit: | 100 | 99 | 97 | 92 | 91 | 82 | 72 | 62 | 50 | 27 |

Tabelle 4

| Absetzvolumen (ml) in Paraffinöl (Typ Pioneer 2660, hochviskos) von der Firma Hansen + Rosenthal | | | | | | | | | |
|---|---|---|---|---|---|---|---|---|---|
| Produkt aus | 10 min | 30 min | 1 h | 2 h | 3 h | 4 h | 8 h | 18 h | 24 h |
| Beispiel 6 | 100 | 98 | 97 | 91 | 87 | 82 | 59 | 45 | 43 |
| Beispiel 7 | 100 | 99 | 98 | 96 | 94 | 93 | 77 | 47 | 43 |
| Beispiel 8 | 100 | 99 | 98 | 94 | 92 | 89 | 71 | 48 | 43 |
| Beispiel 9 | 100 | 99 | 97 | 94 | 91 | 89 | 72 | 44 | 43 |
| Beispiel 10 | 100 | 99 | 98 | 94 | 92 | 90 | 73 | 45 | 43 |
| Beispiel 11 | 100 | 98 | 98 | 93 | 91 | 88 | 67 | 45 | 43 |
| Na-Bentonit: | 100 | 96 | 87 | 61 | 53 | 49 | 44 | 43 | 43 |
| org.mod. Hectorit: | 99 | 98 | 97 | 91 | 85 | 80 | 58 | 45 | 43 |
| ohne Zusatz | 97 | 94 | 84 | 60 | 50 | 47 | 41 | 40 | 40 |

Anhand der nachstehenden Beispiele wird die Erfindung noch näher erläutert.

Beispiel 1: Herstellung von $Al_x Mg_y(OH)_{35-z}(SO_4)_{z/2} \cdot nH_2O$

In einem offenen Rührkessel werden 4 743 g Aluminiumhydroxid-Paste mit 12,73 Gew.% $Al_2O_3$ vorgelegt, mit 8 995 g Wasser verdünnt und danach 2 924,8 g Aluminiumsulfat-Lösung mit 21,54 % $SO_4$ und 4,21 % Al eingerührt. Man läßt über Nacht stehen, damit eventuell vorhandenes $CO_2$-Gas entweichen kann. Es wird 1 336,7 g MgO mit 60,3 % Mg-Gehalt unter Rühren hinzugegeben, wobei eine leichte Erwärmung eintritt. Man läßt noch 2 Stunden weiterrühren und kann dann die Suspension zur weiteren Verarbeitung verwenden.

Analyse: 2,46 % Al, 4,47 % Mg, 3,5 % $SO_4$

Beispiel 2: Herstellung von Natriumcaprylat - $C_7H_{15}COO$ Na

800 g Caprylsäure werden in 7 l Wasser suspendiert und unter Rühren auf 80°C erhitzt. Danach gibt man langsam eine Lösung von 221,8 g NaOH in 500 g Wasser zu und läßt auf Raumtemperatur abkühlen. Die wässrige Lösung dampft man langsam ein und trocknet den Rückstand bei 105°C im Trockenschrank.

Ausbeute: 877 g (95 % d.Th.) weißes Pulver

Beispiel 3: Herstellung von Natriummyristat $C_{13}H_{27}COO$ Na

800 g Myristinsäure werden in 3 l Wasser suspendiert und unter Rühren auf 80 °C erhitzt. Danach gibt man langsam eine Lösung von 140,2 g NaOH in 350 ml Wasser zu und läßt auf Raumtemperatur abkühlen. Dabei fällt das Natriummyristat aus, welches über eine Nutsche abfiltriert wird. Es wird vorsichtig im Trockenschrank bis auf Gewichtskonstanz getrocknet.

Ausbeute: 820 g (89 % d. Th.) weißes Pulver

Beispiel 4: Herstellung von Natriumpalmitat - $C_{15}H_{31}COO$ Na

800 g Palmitinsäure werden in 9 l Wasser suspendiert und unter Rühren auf 80 °C erwärmt. Danach gibt man eine Lösung von 124,8 g NaoH in 350 ml Wasser zu und läßt auf Raumtemperatur abkühlen. Man nutscht ab und trocknet den Rückstand bei 105 °C im Trockenschrank.

Ausbeute: 814 g (94 % d. Th.) weißes Pulver

Beispiel 5: Herstellung von Natriumbehenat - $C_{21}H_{43}COO$ Na

700 g Behensäure werden in 9 000 ml Wasser suspendiert und auf 80 °C erhitzt. Danach gibt man eine Lösung von 83 g NaOH in 350 ml dest. Wasser hinzu. Dabei fällt sofort das Natriumbehenat aus. Man läßt auf Raumtemperatur abkühlen und filtriert den Niederschlag über eine Nutsche ab. Mit je 3 x 200 ml Ethanol wird nachgewaschen und der Rückstand bei 65 °C im Trockenschrank getrocknet.

Ausbeute: 708 g (95 % d.Th.) weißes Pulver

Beispiel 6: Herstellung von $Al_5Mg_{10}(OH)_{31}(CH_3COO)_4$

119,6 g Na-acetat werden in 1 076 g Wasser mittels Rührer suspendiert und zu 2 000 g Al, Mg-hydroxisulfat-Suspension, wie in Beispiel 1 hergestellt, gegeben. Man erwärmt 3 Stunden auf 80 °C, um die Reaktion zu vervollständigen, läßt ab kühlen und filtriert dann das unlösliche Al, Mg-hydroxiacetat ab. Mit Wasser wird so lange nachgewaschen, bis kein Sulfat mehr mit $BaCl_2$-Lösung als $BaSO_4$ nachweisbar ist. Der Filterkuchen wird dann im Trockenschrank bei 105 °C bis auf Gewichtskonstanz getrocknet.

Ausbeute: 395 g (95 % d.Th..)
Beschreibung: weißes, geruchloses, kristallines Pulver
Identifikation: 11,5 % Al i. Tr. (Theorie 11,8 %)
20,9 % Mg i. Tr. (Theorie 21,3 %)
8,3 % C i. Tr. (Theorie 8,4 %)

| Röntgenspektrum (Gerät: Philipps Automated X-Ray Powder Diffraktometer, System APD 15) zu Beispiel 6 | | | |
|---|---|---|---|
| Peak Nr. | 2 - Theta | d ( pm ) | I / I o |
| 1 | 19,870 | 4 46,45 | 82 |
| 2 | 34,170 | 2 62,18 | 56 |
|   | 34,484 | 2 59,86 | 74 |
|   | 35,669 | 2 55,07 | 100 |
|   | 35,669 | 2 51,50 | 77 |
| 3 | 41,400 | 2 17,00 | |
| 4 | 42,500 | 2 13,00 | |
| 5 | 48,200 | 1 89,00 | |
| 6 | 60,309 | 1 53,34 | 49 |
|   | 60,526 | 1 52,84 | 66 |
|   | 61,199 | 1 51,32 | 76 |
|   | 61,633 | 1 50,35 | 60 |
|   | 61,965 | 1 49,63 | 58 |

Beispiel 7: Herstellung von Al- Mg-hydroxi-caprylat $Al_5Mg_{10}(OH)_{31}(C_7H_{15}COO)_4$

242,3 g Na-caprylat (aus Beispiel 2) werden in 2 181 g Wasser mittels Rühren suspendiert und zu 2 000 g Al, Mg-hydroxi-sulfat-Suspension, wie in Beispiel 1 hergestellt, gegeben. Man erwärmt 1 Stunde auf 60°C, um die Reaktion zu vervollständigen, läßt abkühlen und filtriert dann das unlösliche Al, Mg-hydroxi-caprylat ab. Mit Wasser wird so lange nachgewaschen, bis kein Sulfat mehr mit $BaCl_2$-Lösung als $BaSO_4$ nachweisbar ist. Der Filterkuchen wird dann im Trockenschrank bei 105°C bis auf Gewichtskonstanz getrocknet.

Ausbeute: 517 g (96 % d. Th.)
Beschreibung: weißes, geruchloses, kristallines Pulver
Identifikation: 9,0 % Al i. Tr. (Theorie 9,1 %)
16,2 % Mg i. Tr. (Theorie 16,4 %)
25,0 % C i. Tr. (Theorie 26,0 %)

| Röntgenspektrum (Gerät: Philipps Automated X-Ray Powder Diffraktometer, System APD 15) zu Beispiel 7 | | | |
|---|---|---|---|
| Peak Nr. | 2 - Theta | d (pm) | $I / I_o$ |
| 1 | 19,370 | 4 57,84 | 66 |
| 2 | 33,974 | 2 63,65 | 42 |
|   | 34,267 | 2 61,46 | 72 |
|   | 34,506 | 2 59,70 | 88 |
|   | 34,871 | 2 57,07 | 100 |
|   | 35,075 | 2 55,62 | 83 |
|   | 35,608 | 2 51.91 | 62 |
| 3 | 41,4 | 2 17, | |
| 4 | 42,5 | 2 13, | |
| 5 | 48,3 | 1 89, | |
| 6 | 60,652 | 1 525,5 | 67 |
|   | 60,892 | 1 520,1 | 72 |
|   | 61,279 | 1 511,4 | 56 |
|   | 61,714 | 1 501,8 | 46 |

Beispiel 8: Herstellung von Al - Mg-hydroxi-myristat $Al_5 Mg_{10}(OH)_{31}(C_{13}H_{27}COO)_4$

182,5 g Na-myristat (aus Beispiel 3) werden in 1 643 g Wasser mittels Rühren suspendiert und zu 1 000 g Al, Mg-hydroxi-sulfat-Suspension, wie in Beispiel 1 hergestellt, gegeben. Man erwärmt 1 Stunde auf 60° C, um die Reaktion zu vervollständigen, läßt abkühlen und filtriert dann das unlösliche Al, Mg-hydroxi-myristat ab. Mit Wasser wird so lange nachgewaschen, bis kein Sulfat mehr mit $BaCl_2$-Lösung als $BaSO_4$ nachweisbar ist. Der Filterkuchen wird dann im Trockenschrank bei 105° C bis auf Gewichtskonstanz getrocknet.

Ausbeute: 321 g (97 % d. Th.)
Beschreibung: weißes, geruchloses, kristallines Pulver
Identifikation: 7,3 % Al i. Tr. (Theorie 7,4 %)
13,2 % Mg i. Tr. (Theorie 13,4 %)
36,3 % C i. Tr. (Theorie 37,0 %)

| Röntgenspektrum (Gerät: Philipps Automated X-Ray Powder Diffraktometer, System APD 15) zu Beispiel 8 | | | |
|---|---|---|---|
| Peak Nr. | 2 - Theta | d (pm) | $I/I_o$ |
| 1 | 20,909 | 4 24,48 | 100 |
|  | 21,340 | 4 16,00 | 91 |
|  | 21,560 | 4 11,82 | 72 |
| 2 | 33,916 | 2 64,08 | 35 |
|  | 34,089 | 2 62,78 | 49 |
|  | 34,471 | 2 59,95 | 70 |
|  | 35,069 | 2 55,66 | 68 |
|  | 35,524 | 2 52,49 | 51 |
| 3 | 41,4 | 217, |  |
| 4 | 42,5 | 213, |  |
| 5 | 48,2 | 189, |  |
| 6 | 60,318 | 1 53,31 | 28 |
|  | 60,534 | 1 52,82 | 47 |
|  | 61,649 | 1 50,32 | 42 |

Beispiel 9: Herstellung von Al -Mg-hydroxi-palmitat $Al_5Mg_{10}(OH)_{31}(C_{15}H_{31}COO)_4$

405,9 g Na-palmitat (aus Beispiel 4) werden in 3 653 g Wasser mittels Rühren suspendiert und zu 2 000 g Al, Mg-hydroxi-sulfat-Suspension, wie in Beispiel 1 hergestellt, gegeben. Man erwärmt 1 Stunde auf 60° C, um die Reaktion zu vervollständigen, läßt abkühlen und filtriert dann das unlösliche Al, Mg-hydroxi-palmitat ab. Mit Wasser wird so lange nachgewaschen, bis kein Sulfat mehr mit $BaCl_2$-Lösung als $BaSO_4$ nachweisbar ist. Der Filterkuchen wird dann im Trockenschrank bei 105° C bis auf Gewichtskonstanz getrocknet.

Ausbeute: 660 g (94 % d. Th.)
Beschreibung: weißes, geruchloses, kristallines Pulver
Identifikation: 6,8 % Al i. Tr. (Theorie 7,0 %)
12,4 % Mg i. Tr. (Theorie 12,6 %)
39,4 % C i. Tr. (Theorie 39,9 %)

| Röntgenspektrum (Gerät: Philipps Automated X-Ray Powder Diffraktometer, System APD 15) zu Beispiel 9 | | | |
|---|---|---|---|
| Peak Nr. | 2 - Theta | d (pm) | $I / I_o$ |
| 1 | 19,702 | 4 50,21 | 59 |
|   | 21,323 | 4 16,35 | 100 |
| 2 | 31,792 | 2 81,23 | 30 |
| 3 | 34,305 | 2 61,17 | 48 |
|   | 34,615 | 2 58,91 | 76 |
|   | 35,169 | 2 54,95 | 64 |
| 4 | 41,4 | 2 17, | |
| 5 | 42,5 | 2 13, | |
| 6 | 48,3 | 1 89, | |
| 7 | 60,700 | 1 52,44 | 49 |
|   | 61,138 | 1 51,45 | 32 |

Beispiel 10: Herstellung von Al -Mg-hydroxi-stearat $Al_5Mg_{10}(OH)_{31}(C_{17}H_{35}COO)_4$

446,8 g Na-stearat werden in 4 021 g Wasser mittels Rührer suspendiert und zu 2 000 g Al, Mg-hydroxi-sulfat-Suspension, wie in Beispiel 1 hergestellt, gegeben. Man erwärmt 1 Stunde auf 60°C, um die Reaktion zu vervollständigen, läßt abkühlen und filtriert dann das unlösliche Al, Mg-hydroxi-stearat ab. Mit Wasser wird so lange nachgewaschen, bis kein Sulfat mehr mit $BaCl_2$-Lösung als $BaSO_4$ nachweisbar ist. Der Filterkuchen wird dann im Trockenschrank bei 105°C bis auf Gewichtskonstanz getrocknet.

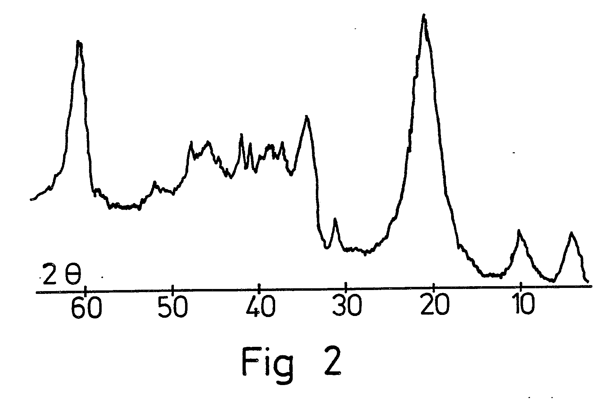

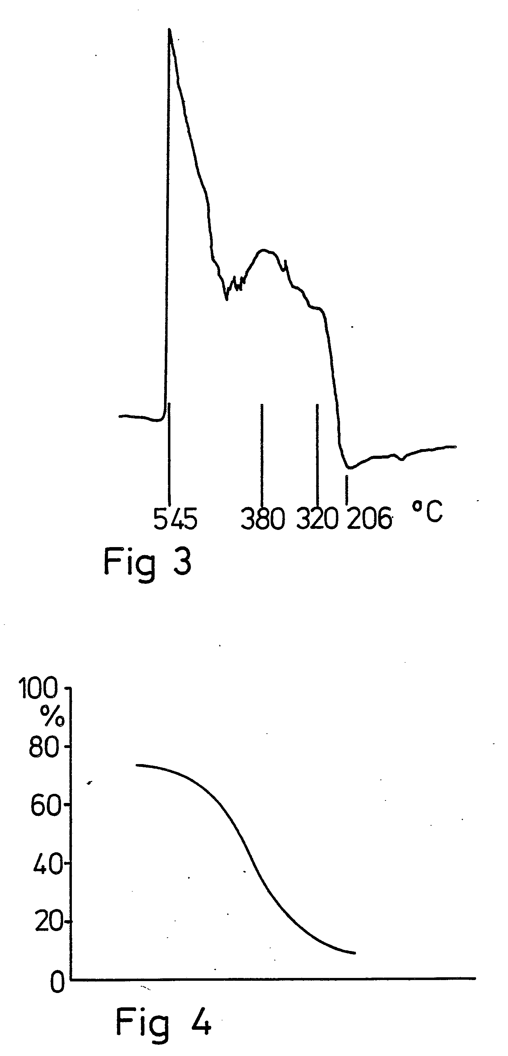

Ausbeute: 738 g (98 % d. Th.)
Beschreibung: weißes, geruchloses, kristallines Pulver
Identifikation: 6,5 % Al i. Tr. (Theorie 6,6 %)
11,7 % Mg i. Tr. (Theorie 11,9 %)
42,2 % C i. Tr. (Theorie 42,4 %)
Die Röntgenaufnahme des Produktes aus Beispiel 10 zeigt Fig.2, die thermische Differential Analyse Fig.3 und die thermographische Analyse Fig. 4.

Fig.1 zeigt die REM-Aufnahme des Produktes aus Beispiel 10.

| Röntgenspektrum (Gerät: Philipps Automated X-Ray Powder Diffraktometer, System APD 15) zu Beispiel 10 | | | |
|---|---|---|---|
| Peak Nr. | 2 - Theta | d (pm) | $I / I_o$ |
| 1 | 19,576 | 4 53,07 | 59 |
|   | 20,794 | 4 26,81 | 91 |
|   | 21,466 | 4 13,59 | 100 |
| 2 | 31,641 | 2 82,53 | 34 |
| 3 | 34,894 | 2 64,25 | 41 |
|   | 34,072 | 2 62,91 | 36 |
|   | 34,401 | 2 60,47 | 47 |
|   | 34,697 | 2 58,32 | 61 |
|   | 35,265 | 2 54,29 | 46 |
|   | 35,5114 | 2 52,56 | 44 |
|   | 35,693 | 2 51,33 | 39 |
|   | 35,873 | 2 50,11 | 34 |
| 4 | 41,4 | 2 17, |   |
| 5 | 42,5 | 2 13, |   |
| 6 | 48,163 | 1 88,77 | 30 |
| 7 | 60,399 | 1 53,13 | 26 |
|   | 60,765 | 1 52,29 | 37 |
|   | 60,974 | 1 51,82 | 35 |
|   | 61,306 | 1 51,08 | 35 |

Beispiel 11: Herstellung von Al-Mg-hydroxi-behenat $Al_5Mg_{10}(OH)_{31}(C_{21}H_{43}COO)_4$

528,6 g Na-behenat (aus Beispiel 5) werden in 4 758 g Wasser mittels Rühren suspendiert und zu 2 000 Al, Mg-hydroxisulfat-Suspension, wie in Beispiel 1 hergestellt, gegeben. Man erwärmt 1 Stunde auf 60°C, um die Reaktion zu vervollständigen, läßt abkühlen und filtriert dann das unlösliche Al, Mg-hydroxi-behenat ab. Mit Wasser wird so lange nachgewaschen, bis kein Sulfat mehr mit $BaCl_2$-Lösung als $BaSO_4$ nachweisbar ist. Der Filterkuchen wird dann im Trockenschrank bei 105°C bis auf Gewichtskonstanz getrocknet.

Ausbeute: 767 g (95 % d. Th.)
Beschreibung: weißes, geruchloses, kristallines Pulver
Identifikation: 5,8 % Al i. Tr. (Theorie 6,0 %)
10,5 % Mg i. Tr. (Theorie 10,7 %)
46,2 % C i. Tr. (Theorie 46,7 %)

11

EP 0 308 646 A2

| Röntgenspektrum (Gerät: Philipps Automated X-Ray Powder Diffraktometer, System APD 15) zu Beispiel 11 | | | |
|---|---|---|---|
| Peak Nr. | 2 - Theta | d (pm) | $I / I_0$ |
| 1 | 19,584 | 4 52,90 | 67 |
|  | 21,049 | 4 21,69 | 82 |
|  | 21,260 | 4 17,56 | 100 |
| 2 | 31,851 | 2 80,72 | 43 |
| 3 | 34,074 | 2 62,89 | 46 |
|  | 34,630 | 2 58,80 | 61 |
|  | 34,857 | 2 57,17 | 73 |
|  | 35,176 | 2 54,90 | 54 |
|  | 35,686 | 2 51,38 | 52 |
| 4 | 41,4 | 2 17, |  |
| 5 | 42,5 | 2 13, |  |
| 6 | 48,3 | 1 89, |  |
| 7 | 60,705 | 1 52,43 | 43 |
|  | 61,982 | 1 49,59 | 28 |

Beispiel 12: Herstellung von Al- Mg-hydroxi-stearat $Al_3 Mg_{13}(OH)_{31}(C_{17}H_{35}COO)_4$

In einem offenen Rührkessel werden 578,2 g Aluminiumhydroxid-Paste mit 12,73 % $Al_2O_3$ vorgelegt, mit 3 151,4 g Wasser verdünnt und danach 796 g Aluminiumsulfat-Lösung welche 4,22 % Al und 21,62 % $SO_4$ enthält, eingerührt. Man läßt über Nacht stehen, damit eventuell vorhandenes $CO_2$-Gas entweichen kann und fügt dann 474 g MgO (im Handel erhältlich) mit 99 % MgO-Gehalt unter Rühren hinzu. Dabei tritt eine leichte Erwärmung ein.

Analyse der Suspension: 1,40 % Al, 5,60 % Mg, 3,46 % $SO_4$

Zu 4 469 g der obigen Suspension fügt man unter Rühren 986 g Na-stearat in 7 000 g Wasser suspendiert hinzu. Man erwärmt eine Stunde auf 60° C, läßt abkühlen und filtriert dann das unlösliche Al, Mg-hydroxi-stearat ab. Mit Wasser wird so lange nachgewaschen, bis kein Sulfat mehr nachweisbar ist. Der Filterkuchen wird wieder in 10 kg Wasser suspendiert und sprühgetrocknet. Die Eintrittstemperatur beträgt 270° C und die Austrittstemperatur 100° C.

Ausbeute: 1 606 g (97 % d. Th.)
Beschreibung: weißes, geruchloses, kristallines Pulver
Identifikation: 3,7 % Al i. Tr. (Theorie 3,9 %)
15,3 % Mg i. Tr. (Theorie 15,4 %)
40,8 % C i. Tr. (Theorie 41,5 %)

Beispiel 13: Herstellung von Al - Mg-hydroxi-stearat $Al_5 Mg_{10}(OH)_{31}(C_{17}H_{35}COO)_4$

In einem offenen Rührkessel werden 1 581 g Aluminiumhydroxid-Paste mit 12.3 % $Al_2O_3$ vorgelegt, mit 3 000 g Wasser verdünnt und danach 975 g Aluminiumsulfat-Lösung (festes Aluminiumsulfat im Handel erhältlich ), welche 4,21 % Al und 21,54 % $SO_4$ enthält, eingerührt. Man läßt über Nacht stehen, damit eventuell vorhandenes $CO_2$-Gas entweichen kann und fügt dann 446 g MgO mit 99 % MgO-Gehalt unter Rühren hinzu. Dabei tritt eine leichte Erwärmung ein.

Analyse der Suspension: 2,41 % Al, 4,40 % Mg, 3,45 % $SO_4$

12

Zu 4 470 g der obigen Suspension fügt man unter Rühren 983 g Natriumstearat in 7 000 g Wasser suspendiert hinzu. Man erwärmt eine Stunde auf 60°C, läßt abkühlen und filtriert dann das unlösliche Al, Mg-hydroxi-stearat ab. Mit Wasser wird so lange nachgewaschen, bis kein Sulfat mehr nachweisbar ist. Der Filterkuchen wird wieder in 10 kg Wasser suspendiert und sprühgetrocknet. Die Eintrittstemperatur beträgt 275°C und die Austrittstemperatur 100°C.

Ausbeute: 1 556 g (95 % d. Th.)
Beschreibung: weißes, geruchloses, kristallines Pulver
Identifikation: 6,5 % Al i. Tr. (Theorie 6,6 %)
11,7 % Mg i. Tr. (Theorie 11,9 %)
42,0 % C i. Tr. (Theorie 42,4 %)
Dichte: 1,19 g/ml


Beispiel 14: Herstellung von Al- Mg-hydroxi-stearat $Al_7Mg_7(OH)_{31}(C_{17}H_{35}COO)_4$

In einem offenen Rührkessel werden 2 086 g Aluminiumhydroxid-Paste mit 12,73 % $Al_2O_3$ vorgelegt, mit 1 825 g Wasser verdünnt und danach 824 g Aluminiumsulfat-Lösung, welche 4,22 % Al und 21,62 % $SO_4$ enthält, eingerührt. Man läßt über Nacht stehen, damit eventuell vorhandenes $CO_2$-Gas entweichen kann und fügt dann 264 g MgO mit 99 % MgO-Gehalt unter Rühren hinzu. Dabei tritt eine leichte Erwärmung ein.

Analyse der Suspension: 3,4 % Al, 3,2 % Mg, 3,7 % $SO_4$
Zu 4 469 g der obigen Suspension fügt man unter Rühren 1 054 g Natrium-stearat in 7 000 g Wasser suspendiert hinzu. Man erwärmt eine Stunde auf 60°C, läßt abkühlen und filtriert dann das unlösliche Al - Mg-hydroxi-stearat ab. Mit Wasser wird so lange nachgewaschen, bis kein Sulfat mehr nachweisbar ist. Der Filterkuchen wird wieder in 10 kg Wasser suspendiert und sprühgetrocknet. Die Eintrittstemperatur beträgt 270°C und die Austrittstemperatur 100°C.

Ausbeute: 1 668 g (96 % d. Th.)
Beschreibung: weißes, geruchloses, kristallines Pulver
Identifikation: 9,2 % Al i. Tr. (Theorie 9,3 %)
8,1 % Mg i. Tr. (Theorie 8,4 %)
41,9 % C i. Tr. (Theorie 42,3 %)


Beispiel 15: Herstellung von Al- Mg-hydroxi-stearat $Al_9Mg_4(OH)_{31}(C_{17}H_{35}COO)_4$

In einem offenen Rührkessel werden 2 881 g Aluminiumhydroxid-Paste mit 12,73 % $Al_2O_3$ vorgelegt, mit 1 126 g Wasser verdünnt und danach 839 g Aluminiumsulfat-Lösung, welche 4,22 % Al und 21,62 % $SO_4$ enthält, eingerührt. Man läßt über Nacht stehen, damit eventuell vorhandenes $CO_2$-Gas entweichen kann und fügt dann 154 g MgO (im Handel erhältlich ) mit 99 % MgO-Gehalt unter Rühren hinzu. Dabei tritt eine leichte Erwärmung ein.

Analyse der Suspension: 4,32 % Al, 4,87 % Mg, 3,85% $SO_4$
Zu 4 469 g der obigen Suspension fügt man unter Rühren 1 098 g Natrium-stearat in 7 000 g Wasser suspendiert hinzu. Man erwärmt eine Stunde auf 60°C, läßt abkühlen und filtriert dann das unlösliche Al, Mg-hydroxi-stearat ab. Mit Wasser wird so lange nachgewaschen, bis kein Sulfat mehr nachweisbar ist. Der Filterkuchen wird wieder in 10 kg Was ser suspendiert und sprühgetrocknet. Die Eintrittstemperatur beträgt 270°C und die Austrittstemperatur 100°C.

Ausbeute: 1 685 g (94 % d. Th.)
Beschreibung: weißes, geruchloses, kristallines Pulver
Identifikation: 12,0 % Al i. Tr. (Theorie 12,1 %)
4,7 g Mg i. Tr. (Theorie 4,9 %)
42,4 % C i. Tr. (Theorie 42,7 %)

Beispiel 16: Herstellung von Al-Mg-hydroxi-stearat $Al_5 Mg_{10}(OH)_{31}(C_{17}H_{35}COO)_4$

In einem 200 l-Rührkessel werden 11,1 kg Aluminiumhydroxid-Paste mit 12,3 % $Al_2O_3$ vorgelegt, mit 30 kg Wasser verdünnt und danach 6,8 kg Aluminiumsulfat-Lösung, welche 4,2 % Al und 21,5 % $SO_4$ enthält, eingerührt. Man rührt 3 Stunden nach und fügt dann 3,1 kg MgO mit 99 % MgO-Gehalt hinzu. Dabei tritt eine leichte Erwärmung ein. Nach weiteren 3 Stunden Rühren gibt man 7,2 kg Natrium-stearat und 49 kg Wasser hinzu. Man rührt noch 2 Stunden und behandelt dann die Suspension mit hohen Scherkräften, um eine homogene Paste zu erhalten. Nach weiterem Rühren (ca. 1 Stunde) filtriert man das unlösliche Al, Mg-hydroxi-stearat über eine Filterprese ab. Mit Wasser wird so lange nachgewaschen, bis kein Sulfat mehr nachweisbar ist. Der Filterkuchen wird in 70 kg Wasser suspendiert und sprühgetrocknet. Die Eintrittstemperatur beträgt 280° C und die Austrittstemperatur 90° C.

Ausbeute: 10,5 kg (92 % der Theorie)
Beschreibung: weißes, geruchloses, kristallines Pulver
Identifikation: 6,5 % Al i. Tr. (Theorie 6,6 %)
11,8 % Mg i. Tr. (Theorie 11,9 %)
42,1 % C i. Tr. (Theorie 42,4 %)

Beispiel 17: Herstellung von Al-Mg-hydroxi-palmitat-stearat $Al_5 Mg_{10}(OH)_{31}(C_{15}H_{31}COO) (C_{17}H_{35}COO)_3$

101 g Na-palmitat (aus Beispiel 4) und 335 g Na-stearat werden in 3 930 g Wasser unter Rühren suspendiert und zu 2 000 g Al-Mg-hydroxi-sulfat-Suspension, wie in Beispiel 1 hergestellt, gegeben. Man homogenisiert mit einem Turrax und rührt 3 Stunden bei Raumtemperatur. Man filtriert ab und wäscht mit dest. Wasser sulfatfrei. Der Filterkuchen wird bei 95° C im Trockenschrank bis auf Gewichtskonstanz getrocknet.

Ausbeute: 693 g (95 % der Theorie)
Beschreibung: weißes, geruchloses, kristallines Pulver
Identifikation: 6,6 Al i. Tr. (Theorie 6,7 %)
11,5 Mg i. Tr. (Theorie 12,0 %)
41,4 C i. Tr. (Theorie 41,8 %)

## Ansprüche

1. Verbindungen der Formel

$$Al_x Mg_y(OH)_{35-z} R_z \cdot n\ H_2O$$

in denen R den Rest R $COO^-$ einer Monocarbonsäure darstellt, wobei die Indices x, y, z folgende Bedingungen erfüllen:
$3 \leq x \leq 9$
$4 \leq y \leq 13$
$3 \leq z \leq 5$ und $3x + 2y = 35$ .

2. Verbindungen nach Anspruch 1, dadurch gekennzeichnet, daß x = 5, y = 10 und z = 4 ist.

3. Verbindungen nach Anspruch 1 und 2, dadurch gekennzeichnet, daß der Rest R $COO^-$ 2 bis 22 Kohlenstoffatome enthält.

4. Verbindungen nach den Ansprüchen 1 bis 3, dadurch gekennzeichnet, daß die den Rest R $COO^-$ enthaltende Carbonsäure ein technisches Gemisch von aliphatischen Monocarbonsäuren ist, insbesondere Monocarbonsäuren mit 16 und 18 Kohlenstoffatomen.

5. Verfahren zur Herstellung der Verbindungen nach Anspruch 1, dadurch gekennzeichnet, daß eine wässrige Suspension der Verbindung $Al_x Mg_y(OH)_{35-z}(SO_4)_{z/2} \cdot nH_2O$, in der die Indices

$3 \leq x \leq 9$
$4 \leq y \leq 13$
$3 \leq z \leq 5$ und $3x + 2y = 35$ ist

mit einem festen oder in Wasser suspendierten Alkalisalz einer Monocarbonsäure, in welcher der Rest R COO⁻ 2 bis 22 Kohlenstoffatome enthält, bei Temperaturen zwischen 20 bis 100° C, bevorzugt zwischen 20 und 60° C, unter Rühren umgesetzt wird.

6. Verfahren nach Anspruch 5, dadurch gekennzeichnet, daß die Umsetzung der beiden Reaktanten unter Einwirkung von Scherkräften durchgeführt wird.

7. Verfahren nach den Ansprüchen 5 und 6, dadurch gekennzeichnet, daß der Feststoffanteil durch Filtration aus der Suspension abgetrennt wird.

8. Verfahren nach Anspruch 7, dadurch gekennzeichnet, daß der Filterkuchen sulfatfrei gewaschen wird.

9. Verfahren nach den Ansprüchen 5 bis 8, dadurch gekennzeichnet, daß der Filterkuchen bei Temperaturen zwischen 60 bis 130° C, bevorzugt zwischen 80 bis 110° C getrocknet wird.

10. Verfahren nach den Ansprüchen 5 bis 9, dadurch gekennzeichnet, daß der sulfatfreie Filterkuchen in Wasser resuspendiert wird und diese Suspension sprühgetrocknet wird, wobei die Eintrittstemperatur $T_E$ = 250 bis 350° C, bevorzugt 270 bis 300° C, und die Austrittstemperatur $T_A$ 80 bis 130° C, bevorzugt 90 bis 110° C betragen.

Fig. 1

Fig 2

545    380 320 206   °C

## Fig 3

100
%
80
60
40
20
0

## Fig 4